(19) **Europäisches Patentamt**
**European Patent Office**
**Office européen des brevets**

(11) **EP 3 463 571 B1**

(12) **FASCICULE DE BREVET EUROPEEN**

(45) Date de publication et mention de la délivrance du brevet:
**10.06.2020 Bulletin 2020/24**

(21) Numéro de dépôt: **17721151.3**

(22) Date de dépôt: **04.05.2017**

(51) Int Cl.:
**A61N 5/10** $^{(2006.01)}$

(86) Numéro de dépôt international:
**PCT/EP2017/060618**

(87) Numéro de publication internationale:
**WO 2017/207204 (07.12.2017 Gazette 2017/49)**

(54) **PROCEDE ET DISPOSITIF DE CARACTERISATION D'UN FAISCEAU DE CORPUSCULES**

VERFAHREN UND VORRICHTUNG ZUR CHARAKTERISIERUNG EINES STRAHLS VON KORPUSKELN

METHOD AND DEVICE FOR CHARACTERISING A BEAM OF CORPUSCLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.05.2016 FR 1654840**

(43) Date de publication de la demande:
**10.04.2019 Bulletin 2019/15**

(73) Titulaire: **Commissariat à l'Énergie Atomique et aux Énergies Alternatives 75015 Paris (FR)**

(72) Inventeurs:
 • **BARAT, Eric**
  **91470 Limours (FR)**
 • **LAZARO, Delphine**
  **91430 Vauhallan (FR)**
 • **CHABERT, Isabelle**
  **75014 Paris (FR)**

(74) Mandataire: **Dudouit, Isabelle et al Marks & Clerk France Conseils en Propriété Industrielle Immeuble " Visium " 22, avenue Aristide Briand 94117 Arcueil Cedex (FR)**

(56) Documents cités:
**US-B1- 6 535 837**

 • Yves Rozenholc ET AL: "Combining Regular and Irregular Histograms by Penalized Likelihood", , 23 novembre 2009 (2009-11-23), XP055336489, Extrait de l'Internet: URL:https://www.statistik.tu-dortmund.de/fileadmin/user_upload/SFB_823/discussion_papers/2009/31_09_rozenholc_mildenberger_gather.pdf [extrait le 2017-01-18] cité dans la demande
 • FIX MICHAEL ET AL: "Monte Carlo source model for photon beam radiotherapy: photon source characteristics", MEDICAL PHYSICS, AIP, MELVILLE, NY, US, vol. 31, no. 11, 28 octobre 2004 (2004-10-28), pages 3106-3121, XP012075083, ISSN: 0094-2405, DOI: 10.1118/1.1803431 cité dans la demande

EP 3 463 571 B1

**Description**

L'objet de l'invention concerne un procédé et un dispositif permettant la caractérisation d'un faisceau de particules, au sein d'un accélérateur de particules, par exemple, afin notamment de déterminer la dose déposée dans un milieu par les corpuscules émis.

Elle s'applique, par exemple pour caractériser les paramètres physiques d'un faisceau d'irradiation de rayons X ou d'électrons modélisés par une simulation de type Monte Carlo. Elle est utilisée, par exemple, dans le domaine de la radiothérapie, de l'imagerie médicale utilisant les rayons X afin de calculer les doses utilisées.

Les logiciels de calcul de la dose reçue par un patient au cours d'un traitement de radiothérapie ou lors d'un examen d'imagerie par rayons X nécessitent une connaissance des propriétés physiques du faisceau utilisé pour l'irradiation. Par propriétés physiques, on entend l'énergie, la direction et la position des particules présentes dans ce faisceau. Le calcul de la dose délivrée repose de plus en plus sur des méthodes de calcul de type Monte-Carlo, MC, en raison de leur supériorité à délivrer une information précise, quelques que soient les situations cliniques rencontrées. Avec ces méthodes MC, on réalise d'ordinaire le calcul de dose en deux étapes : la première consiste à calculer un fichier d'espace des phases (noté PSF), qui contient la description physique du faisceau d'irradiation délivré par le dispositif médical thérapeutique, en radiothérapie, ou diagnostique en imagerie médicale par rayons X. Dans une seconde étape, on utilise cet espace des phases pour réaliser le calcul de dose proprement dit, dans une scène géométrique qui décrit par exemple le patient à soigner.

Dans une approche Monte Carlo, le faisceau d'irradiation est représenté par un échantillon aléatoire, i.e., une collection de particules générées suivant la distribution de probabilité caractérisant le faisceau. Le problème technique posé porte sur le fichier d'espace des phases. Ce fichier stocke toutes les particules appartenant au faisceau et possède la structure suivante : les propriétés de chaque particule du faisceau sont stockées sur une ligne. Ce choix de représentation entraîne trois limitations techniques :

1 - la taille du fichier : pour que le calcul de dose par méthode MC soit représentatif sur un plan statistique, il est nécessaire de stocker un nombre d'échantillons important, par exemple plusieurs millions. Le fichier peut donc atteindre plusieurs centaines de giga-octets, voire plusieurs téraoctets, et devient donc difficilement gérable lorsqu'il est intégré à un logiciel de calcul de dose,

2 - le nombre limité de particules stockées dans le fichier peut être à l'origine d'erreurs systématiques, à l'image d'un bruit, sur la représentation du faisceau. Ces erreurs se propagent dans le calcul de dose ultérieur ou de biais si le fichier est lu et utilisé trop souvent lors du calcul de dose,

3 - le caractère non flexible de ce mode de représentation : le fichier d'espace des phases décrit l'état du faisceau à un instant donné et correspond à un paramétrage donné. Si le paramétrage du faisceau change, même légèrement, le fichier doit être de nouveau calculé. Ce mode de représentation est donc peu propice pour prendre en compte les légères variations physiques existantes entre deux faisceaux issus du même dispositif d'irradiation.

Il existe donc un besoin de fournir un procédé et un système permettant de stocker de façon plus compacte les caractéristiques physiques d'un faisceau d'irradiation, de générer le nombre de particules nécessaires au calcul de dose, sans limite intrinsèque, de manière flexible et rapide.

L'article de C.M. Ma, B.A. Faddegon, D.W.O. Rogers et T.R. Mackie intitulé Accurate characterization of Monte Carlo calculated electron beams for radiotherapy, Medical Physics, 24, pages 401-416, 1997, décrit un accélérateur linéaire médical délivrant un faisceau de photons de haute énergie (quelques Méga-Volts, MV). Trois groupes particuliers de photons se distinguent : les photons provenant de la cible de conversion sur laquelle frappent les électrons incidents, les photons ayant diffusé dans le collimateur primaire et les photons ayant diffusé dans le cône égalisateur. Plusieurs stratégies ont été proposées pour construire les modèles de sources virtuelles :

(a) soit à partir des données contenues dans les espaces des phases pré-calculées par modélisation MC du faisceau d'irradiation,
(b) soit à partir de données expérimentales.

Le document de A.E. Schach von Wittenau, et al, intitulé Correlated Histogram Representation of Monte Carlo Derived Medical Accelerator Photon-Output Phase Space, Medical Physics, 26 (7), pages 1196-1211, 1999, décrit l'utilisation d'histogrammes multidimensionnels corrélés qui décrivent les corrélations entre les variables physiques décrivant l'état du faisceau, à savoir l'énergie, la position et la direction de chaque particule. Le nombre de ces variables est différent suivant le repère choisi et le modèle de source à construire. Il y a un histogramme corrélé par source élémentaire. L'avantage de cette approche est de conserver au sein des histogrammes les corrélations existantes entre toutes les variables stockées dans le fichier d'espace des phases, ce qui permet une représentation plus compacte.

La publication de M. Fix, et al, intitulé Monte Carlo Source Model for Megavoltage Photon-Beam Radiotherapy:

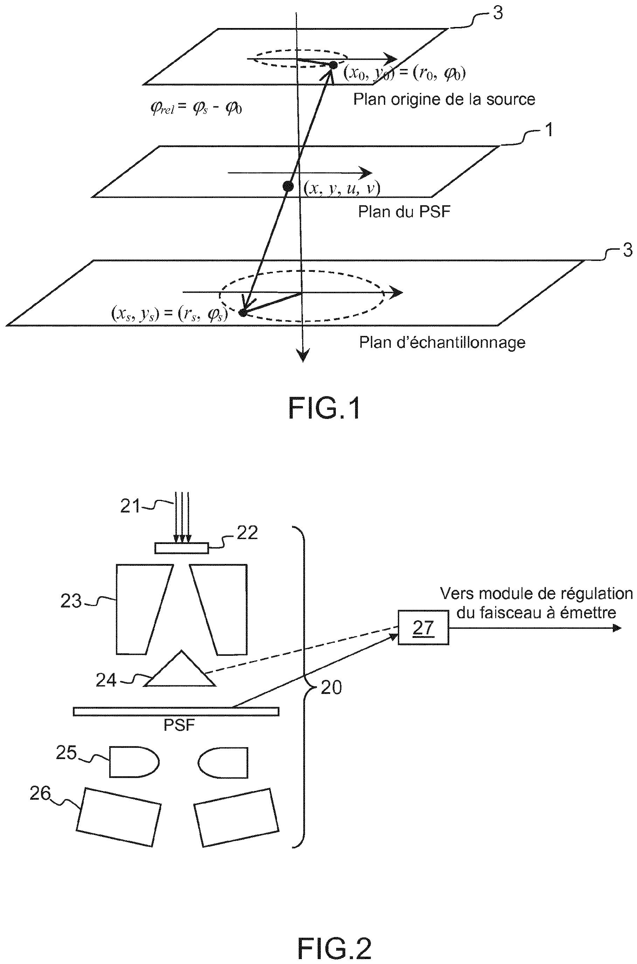

Photon Source Characteristics, Medical Physics, 31 (11), pages 3106-3121, 2004, divulgue un système dans lequel sont représentés les trois composants physiques essentiels dans la tête de l'accélérateur linéaire médical : la cible, le collimateur primaire, et le cône égalisateur. La population de photons issus de ces trois composants possède des caractéristiques en position, en direction et en énergie bien particulières. Le procédé décrit va chercher à représenter avec le plus de précision possible le comportement physique de ces différentes populations de photons, à différentes énergies (6 et 18 MV). Les auteurs réalisent donc un calcul de l'espace des phases par méthode MC à l'aide d'un code MC appelé BEAMnrc et connu de l'homme du métier dans le domaine de la radiothérapie, pour un accélérateur linéaire médical donné. Le fichier d'espace des phases est ensuite stocké au-dessus des systèmes de collimation secondaires (mâchoires et collimateur multi-lames). Un premier tri des particules est réalisé pour séparer les photons des électrons, ces derniers étant stockés dans un fichier à part. Les photons constituent la population majoritaire de l'espace des phases et sont eux-mêmes triés en trois groupes, selon que leur dernière interaction a eu lieu dans la cible, le collimateur primaire ou le cône égalisateur. Les auteurs définissent ensuite un repère (cf Figure 1), dans lequel les positions et directions des photons vont être représentées. Dans ce repère, trois plans représentés sur la Figure 1 servent à mettre en place le modèle pour chaque source élémentaire.

Le premier plan 1, noté « plan du PSF », est celui dans lequel est enregistré l'espace des phases lors du calcul MC préliminaire. Le second plan 2 est celui du plan d'échantillonnage : il est positionné de manière à être confondu avec le plan de l'isocentre, mais peut être a priori localisé ailleurs. L'altitude, par rapport au plan du PSF, du troisième plan 3 dépend de la source élémentaire modélisée, car il correspond au plan de sortie du composant qu'elle représente. Il est appelé « plan origine ».

Les coordonnées de chaque photon stockées dans le fichier d'espace des phases, au niveau du plan du PSF, sont notées (x, y). Les coordonnées sont projetées dans le plan origine et le plan d'échantillonnage par « lancer de lumière » ou en anglo-saxon « ray-tracing », en utilisant la direction des photons (u,v), elles sont également stockées dans le fichier d'espace des phases. Les coordonnées polaires de la particule dans le plan origine et dans le plan d'échantillonnage sont notées $(r_0, \varphi_0)$ et $(r_s, \varphi_s)$, respectivement. La différence des angles $\varphi_s$ et $\varphi_0$ est notée $\varphi_{rel}$.

L'espace des phases de chacune des trois sources élémentaires est alors décrit par une fonction à plusieurs dimensions qui peut être modélisée par les équations 1 ou 2 (ci-dessous), où fi représente la position et la direction des particules appartenant à la source élémentaire i et gi son énergie. Ces fonctions sont décrites par des histogrammes qui dépendent du modèle choisi.

$$F_i(x, y, v, E) = f_i\big(r_s, \varphi_s, r_0(r_s), \varphi_0(r_s)\big) . g_i\big(E(r_s)\big) \qquad \text{(équation 1)}$$

$$F_i(x, y, v, E) = f_i\big(r_s, \varphi_s, r_0(r_s), \varphi_0(r_s, r_0)\big) . g_i\big(E(r_s, r_0)\big) \qquad \text{(équation 2)}$$

E : énergie

A chacune de ces équations correspond en fait un modèle de source. Le premier modèle, correspondant à l'équation 1, est inspiré de celui proposé par Deng et al, Photon Beam Characterization and Modelling for Monte Carlo Treatment Planning, Physics in Medicine and Biology, 45 (2): pages 411-427, 2000. Ce modèle de source compact, abrégé MSC par la suite, ne prend en compte que la dépendance de $r_0$, E, et $\varphi_0$ avec $r_s$. L'angle $\varphi_{rel}$ traduit la direction de la particule, c'est donc lui qui est utilisé dans le modèle. On construit tout d'abord un histogramme principal qui correspond à la distribution de la variable $r_s$. Pour chacun des $n_{tot}$ canaux de l'histogramme, on détermine les histogrammes secondaires associés en $r_0$, E, et $\varphi_{rel}$. Ces histogrammes représentent les distributions en $r_0$, E, et $\varphi_{rel}$ des particules p qui ont une position radiale $r_s$, comprise dans le n$^{ième}$ canal de l'histogramme principal. On a donc autant d'histogrammes secondaires en $r_0$, E, et $\varphi_{rel}$ que de canaux dans l'histogramme principal en $r_s$, soit $n_{tot}$. Le second modèle (équation 2) développé par Fix, propose une amélioration par rapport au premier modèle, qui consiste à prendre en compte une dépendance supplémentaire, celle de l'énergie E et de l'angle $\varphi_0$ avec $r_0$. Comme pour le premier modèle, on construit un histogramme principal qui correspond à la distribution de la variable $r_s$. On construit également un histogramme secondaire en $r_0$ pour chaque canal de l'histogramme principal. Pour chacun des $m_{tot}$ canaux des $n_{tot}$ histogrammes secondaires en $r_0$, on construit les histogrammes des variables E, et $\varphi_{rel}$. Ces histogrammes décrivent respectivement la distribution en énergie et en angle $\varphi_{rel}$ des particules p dont les positions radiales $r_s$, et $r_0$, sont comprises dans le m$^{ième}$ canal de l'histogramme secondaire en $r_0$ correspondant au n$^{ième}$ canal de l'histogramme principal en $r_s$. On a donc un histogramme en $r_s$ de $n_{tot}$ canaux, $n_{tot}$ histogrammes en $r_0$ composés de $m_{tot}$ canaux, et enfin $n_{tot} \times m_{tot}$ histogrammes en E, et $\varphi_{rel}$. L'utilisation des modèles se fait par des tirages aléatoires successifs dans les différents histogrammes.

La précision de chacun des deux modèles est évaluée source par source, grâce à la comparaison de distributions de dose dans l'eau obtenues avec les modèles de source et par simulations MC conventionnelles. Plusieurs tailles de champ (1 x 1 cm$^2$ à 30 x 30 cm$^2$) et plusieurs distances source-profondeur dans la cuve à eau (50, 75, 100 et 200 cm) sont testées. La taille des voxels est de 0,4 x 0,4 x 0,4 cm$^3$. Le code utilisé est alors DOSXYZnrc connu de l'homme du métier. La simulation MC est ici considérée comme étant la référence. Cette étude est l'une des seules à proposer une validation de MSC pour des distances source-profondeur supérieures à 200 cm, ce qui est intéressant puisqu'il s'agit de la distance possible des imageurs embarqués. Le modèle 1 produit de bons résultats pour une distance de 50 cm, la supériorité du modèle 2 permet de mieux reproduire le faisceau réel que le modèle 1.

Le brevet US 6,535,837 décrit une méthode pour représenter de façon compacte l'espace des phases afin de caractériser un faisceau de corpuscules pour le calcul de la dose délivrée au patient dans un système de radiothérapie.

L'invention concerne un procédé pour estimer les paramètres caractéristiques physiques ou variables d'un faisceau de corpuscules destiné à être émis vers un corps ou un objet et pour déterminer la dose déposée par le faisceau émis, les corpuscules étant rangés dans un fichier d'espace des phases, comportant au moins les étapes suivantes :

- Collecter les corpuscules présents dans un faisceau ayant traversé un collecteur et un filtre, ayant interagi ou non avec eux,
- Déterminer une partition d'un support $\Omega$ de chaque variable sous forme d'un ensemble $I$ d'intervalles consécutifs et contigus pour le classement des corpuscules du fichier PSF en énergie, position et direction, la réunion de ces intervalles pour chaque variable du faisceau constituant un canal multi-varié,

Le procédé est caractérisé en ce qu'il comporte les étapes suivantes :

- Découper ledit support $\Omega$ en un nombre $D_{max}$ de micro-canaux réguliers de taille donnée $\delta$ et calculer les vraisemblances correspondant aux intervalles possibles par concaténation contigüe des micro-canaux, retenir la meilleure partition $I$ de $\Omega$,
- Définir un maillage dans chaque direction de l'espace des phases en maximisant la vraisemblance de la réalisation d'une variable dans un intervalle en introduisant un « a priori », afin d'obtenir une partition régularisée de l'espace des phases,
- Estimer la distribution de probabilité de l'espace des phases pour la partition régularisée,
- Calculer les caractéristiques physiques du faisceau de corpuscules, les comparer à des données prévues et déterminer les doses avant d'émettre le faisceau.

L'invention concerne aussi un dispositif pour estimer les paramètres caractéristiques physiques d'un faisceau de corpuscules destiné à être émis vers un corps ou un objet et pour déterminer la dose à émettre, les corpuscules étant rangés dans un fichier d'espace des phases, caractérisé en ce qu'il comporte au moins les éléments suivants :

- Un collecteur des corpuscules émis dans un faisceau,
- Un processeur adapté à exécuter les étapes du procédé selon l'invention.

Le dispositif est par exemple, un dispositif médical et les corpuscules les rayons X pour lesquels on souhaite déterminer la dose à émettre.

D'autres caractéristiques et avantages de la présente invention apparaîtront mieux à la lecture de la description d'exemples donnés à titre illustratif et nullement limitatifs annexée des figures qui représentent :

- Figure 1, une représentation des plans choisis par l'art antérieur pour la représentation d'histogrammes de distribution de particules,
- Figure 2, un exemple d'architecture d'un système accélérateur de particules (dit aussi accélérateur linéaire médical),
- Figure 3, une représentation des densités de probabilités de variables et leur densité,
- Figure 4, une représentation de l'application du mécanisme d'arbre de Polya.

Le procédé et le système selon l'invention, pour caractériser et déterminer une dose déposée par un faisceau de corpuscules, reposent notamment sur la mise en œuvre d'un modèle de source compact (MSC) basé sur des histogrammes multi-variés de la distribution des particules dans un faisceau, de dimension supérieure à trois prenant en compte toutes les corrélations possibles entre les variables de l'espace de phases. La taille mémoire à utiliser pour stocker par exemple les caractéristiques du faisceau est réduite selon le procédé de MSC. Tout en réduisant la taille mémoire nécessaire, le modèle de source contient toute l'information présente dans le PSF, en particulier les corrélations entre toutes les variables nécessaires à la caractérisation du faisceau (énergie, position, direction des particules). La réduction de la taille mémoire facilite l'intégration logicielle du modèle et accélère sa manipulation par rapport au PSF car le MSC peut être entièrement stocké en une fois en mémoire vive de l'ordinateur. Le procédé selon l'invention consiste notamment à utiliser un maillage irrégulier des canaux, optimal au sens d'un critère statistique de type maximum a posteriori et une technique de lissage des histogrammes produits agissant comme un débruitage de ces derniers (histogrammes qui comportent les paramètres physiques caractéristiques d'un faisceau, à savoir l'énergie, la position et la direction des particules contenues dans un faisceau). D'autre part, la mise en œuvre du procédé permet d'utiliser un PSF pour la création du MSC moins volumineux que si le calcul de dose était effectué en l'absence de MSC, soit directement à partir de ce dit PSF. Le procédé permet ainsi de réduire le temps de simulation requis pour la constitution du PSF et augmente la précision des calculs de dose.

La figure 2 donne un exemple d'architecture d'un LINAC comprenant un module de mise en œuvre des étapes du procédé selon l'invention.

Le système accélérateur de particules (dit aussi accélérateur linéaire médical) 20 comporte une source primaire de particules 21, typiquement des électrons de haute énergie (MV), issus d'un canon à électrons et accélérés dans un tube où règne le vide (appelé section accélératrice) par l'onde électromagnétique produite par un électro-aimant. Le faisceau d'électrons primaires rencontre ensuite une cible de conversion en matériau lourd 22, produisant ainsi un faisceau divergent et multi-énergies de rayons X à haute énergie par interaction (Bremsstrahlung) des électrons avec la cible. Le faisceau de rayons X est ensuite mis en forme, en termes de géométrie et d'intensité, à l'aide de différents systèmes de collimation, collimateur primaire 23, et d'égalisation, filtre égalisateur 24. On retrouve ensuite d'autres éléments de collimation, par exemple des collimateurs multi-lames 25 et des mâchoires 26, qui permettent de conformer le faisceau d'irradiation pour chaque traitement, individuellement. Les composants 21, 22, 23 et 24 constituent la partie haute de la tête de l'accélérateur, qui reste fixe pour toutes les irradiations. Sur les nouvelles générations d'accélérateurs de particules, le composant 24 tend à disparaître. Les composants 25 et 26 sont mobiles et leur placement, complexe, dépend du traitement à délivrer et est calculé au cas par cas. Le PSF peut alors être calculé en sortie de la partie haute du système accélérateur de particules (sortie du composant 24) en utilisant un processeur 27 comportant un algorithme de traitement adapté à exécuter les étapes du procédé selon l'invention et fournir le MSC. Le processeur 27 comporte également un module de calcul de dose utilisant le MSC. La dose calculée peut alors, par exemple, être utilisée pour optimiser le faisceau de corpuscules émis par le dispositif médical pour le traitement des tumeurs.

Les faisceaux de rayons X utilisés en imagerie médicale sont de plus basse énergie (kV) et issus d'un tube à rayons X. Dans un tube à rayons X, une cible en matériau lourd (appelée anode) est bombardée par un faisceau d'électrons accélérés dans le vide, ces électrons étant obtenus en chauffant un filament appelé cathode (effet thermo-ionique) et en étant accélérés par une forte différence de potentiel. Des filtres additionnels (cuivre, aluminium) sont utilisés pour mettre en forme le spectre en énergie des photons issus de la cible.

Le fichier PSF représente un échantillon statistique des particules ou corpuscules représentant la distribution des particules dans le faisceau. Le procédé consistant à déterminer les caractéristiques du faisceau selon un histogramme multidimensionnel (ou multi-variés) consiste à ranger chaque particule présente dans le PSF dans un canal multi-varié. Ainsi, pour chaque variable intervenant dans la caractérisation du faisceau (énergie, position, direction des particules), des intervalles consécutifs et contigus doivent être déterminés pour permettre le classement des particules ou corpuscules. La réunion de ces intervalles pour chaque variable du faisceau constitue un canal multi-varié. Le modèle de source consistant à représenter la distribution des particules du faisceau selon un histogramme multi-varié revient donc à considérer que la densité de probabilité de cette distribution est uniforme sur chaque canal considéré.

Le procédé selon l'invention va utiliser deux étapes principales consistant pour la première à réaliser un maillage irrégulier, adaptatif et régularisé de l'espace multidimensionnel dans lequel le faisceau de particules est observé, pour la deuxième à utiliser une technique d'estimation de densité de probabilité qui peut être interprétée comme un lissage des histogrammes corrélés obtenus après accumulation des particules dans les canaux issus du maillage. L'issue de ces deux étapes permettra de déterminer la dose déposée par les particules émises.

Le processeur 27 va donc être adapté à exécuter les étapes du procédé détaillées ci-après.

L'optimisation de la largeur des intervalles est traitée indépendamment pour chaque variable intervenant dans la source compacte : énergie, rayon ou positions en l'absence de symétrie radiale, angle polaire, angle azimutal. Le maillage multidimensionnel est ainsi séparable en chaque variable intervenant dans le même modèle. Dans ce qui suit, la technique est présentée pour une des variables considérées (énergie, position, directions).

La première étape est de créer des canaux irréguliers pour le rangement des particules du PSF dont les largeurs sont optimisées par un critère du maximum de vraisemblance, par exemple. Ceci est réalisé en appliquant une modélisation probabiliste des données stockées sous forme d'histogramme pour chaque variable précitée intervenant dans la caractérisation du faisceau.

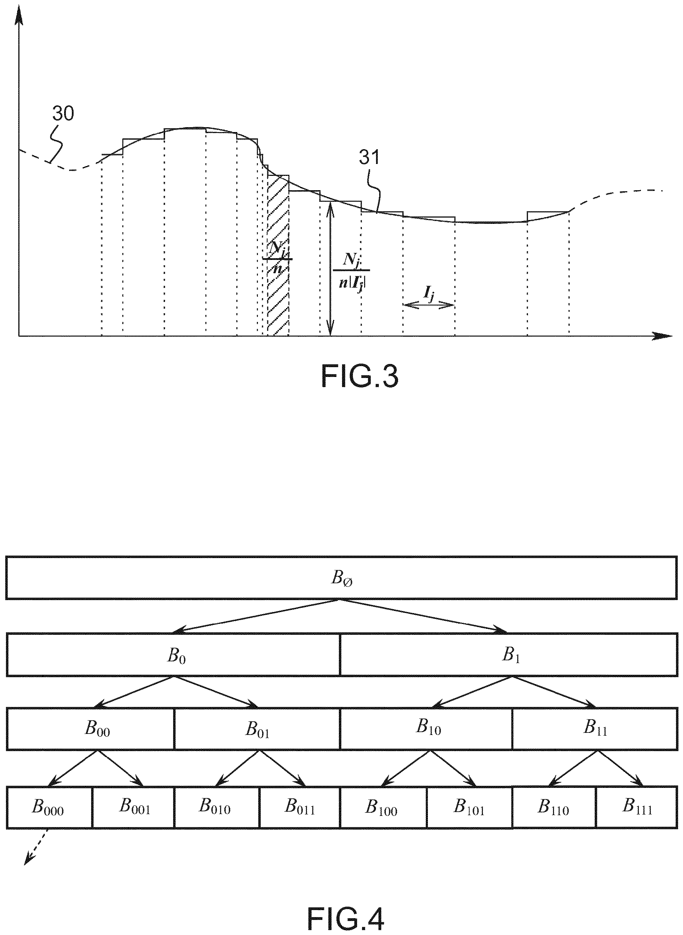

La figure 3 schématise la densité de probabilité théorique d'une variable arbitraire de l'espace des phases, 30. L'estimation de cette distribution par le MSC pris dans la dimension de la variable est présentée sous la forme d'histogramme normalisé, 31. La densité du MSC est constante sur un canal. Le maillage représenté dans cet exemple est irrégulier et représentatif du résultat de l'algorithme adaptatif utilisé.

La log-vraisemblance, notée $L_1$, est dans le cas d'histogrammes irréguliers contenant les échantillons d'une des variables caractérisant les particules du PSF définie par l'équation (1) :

$$L_1 \triangleq log\big(P(X|I)\big) \qquad (1)$$

où *X* est une variable aléatoire décrivant la répartition des particules du PSF au sein de l'ensemble des intervalles *I* définissant une partition du support Ω de la variable. Ce support est partitionné en une succession de *D* canaux dont le *j*<sup>ème</sup> a pour largeur *I<sub>j</sub>*. Dans le cas d'une source à 4 variables, *D* peut être typiquement fixé à 64. Si *n* et *N<sub>j</sub>* désignent respectivement le nombre total de particules totales du faisceau et le nombre de particules dans l'intervalle *I<sub>j</sub>*, alors $X = \{N_1, N_2, ..., N_D\}$, $I = \{I_1, I_2, ..., I_D\}$ et $P(X|I)$, la probabilité de la réalisation de la variable *X* sachant la collection d'intervalles *I* s'écrit :

$$P(X|I) = \prod_{j=1}^{D} \left( \frac{N_j}{n|I_j|} \right)^{N_j} \tag{2}$$

Cette expression est caractéristique du modèle de source proposé pour lequel la densité de probabilité des variables caractérisant le faisceau est considérée constante par canal et revient à la probabilité d'avoir *N<sub>j</sub>* particules du PSF dans l'intervalle *I<sub>j</sub>*.

L'histogramme empirique associé à la partition en intervalles *I* présente une densité de probabilité estimée pour le canal *j* (de largeur *I<sub>j</sub>*) égale à $\frac{N_j}{n|I_j|}$. Dès lors, la probabilité d'observer *N<sub>j</sub>* particules indépendantes venant de ce canal est $\left( \frac{N_j}{n|I_j|} \right)^{N_j}$. Les *D* canaux étant indépendants, on obtient l'expression de *P(X|I)*, la probabilité d'observer N particules indépendantes venant de l'ensemble des canaux.

On en déduit l'expression de la log-vraisemblance des intervalles en fonction des particules du PSF :

$$L_1 = \sum_{j=1}^{D} N_j . log \left( \frac{N_j}{n|I_j|} \right) \tag{3}$$

L'expression de (-*L*<sub>1</sub>) peut être vue comme une fonction de coût à minimiser. On utilise par exemple un critère quantitatif qui permet de contrôler l'approximation faite en représentant l'espace des phases par un histogramme. Le maillage retenu est celui qui est optimal au sens de ce critère.

L'algorithme présenté ci-dessous s'appuie sur la programmation linéaire pour maximiser le critère *L*<sub>1</sub> donné par exemple dans la publication de Rozenholc et al., Combining regular and irregular histograms by penalized likelihood, Computational Statistics and Data Analysis, (2010), 54, 3313-3323. Le processus de maximisation suit les trois étapes :

(a) Le support Ω est découpé en un grand nombre *D<sub>max</sub>* (typiquement *D<sub>max</sub>* = 2048) de micro-canaux réguliers de tailles égales à δ. Les vraisemblances correspondant à tous les intervalles possibles constitués par la concaténation contigüe des micro-canaux sont calculées ;

(b) Ces micro-canaux sont associés selon tous les regroupements contigus possibles, à chaque fois de manière à produire *D* canaux ;

(c) La meilleure association, et donc la meilleure partition *I* de Ω, est celle qui maximise *L*<sub>1</sub>. C'est ce maillage qui sera retenu dans le procédé selon l'invention.

Une implémentation possible de ces trois étapes est présentée ci-dessous

*Étape (a)* :

Pour *i* = 0 ... *D<sub>max</sub>* D<sub>max</sub> : nombre de micro-canaux donnés initialement Initialiser *M* = 0
Pour *j* = *i* + 1 ... *D<sub>max</sub>* boucle sur les micro-canaux *j* supérieurs à *i*.

Calculer *M* = *M* + *m<sub>i</sub>* où *m<sub>l</sub>* représente le nombre de particules dans le micro-canal *l*.

Calculer $p_1(i,j) = \frac{M}{n} \log \frac{M}{n(j-i)\delta}$ probabilité de regrouper les micro-canaux de *i* à *j* pour former un canal.

*Étape (b) :*

Initialiser pour $j$ = 1 ... $D_{max}$ boucle sur les j micro-canaux
$q(j) = p_1(0,j)$
Pour $i$ = 2...$D$ (nombre de canaux du PSF)

Pour $j = i...D_{max}$ boucle sur les j micro-canaux
Initialiser $S = -\infty$
Pour $d = i - 1...j - 1$

$$s = q(d) + p_1(d,j)$$

Si $s > S$
$S = s$
$H(i,j) = d$

$$p(i,j) = q\big(H(i,j)\big) + p_1(H(i,j),j)$$

Pour $j = i ... D_{max}$ boucle sur les j micro-canaux
$q(j) = p(i,j)$

*Étape (c) :*

Initialiser $L = D_{max}$ avec $D_{max}$ : nombre de micro-canaux donnés initialement Pour $j = D ...1$
$L = c(H(j,L[1]),L)$ où $L[1]$ représente la première coordonnée du vecteur $L$
et $c(u, L)$ la concaténation des vecteurs $u$ et $L$,
on obtient le partitionnement des intervalles.
$L$ est ainsi le vecteur contenant les indices de micro-canaux correspondant aux frontières de la partition optimale au sens ML constituée de $D$ canaux.
L'étape suivante consiste à générer un maillage régularisé et adaptatif afin de gérer plus facilement les histogrammes corrélés de grandes dimensions et donc de gagner en temps de calcul. Ce maillage va notamment permettre de décrire aussi précisément que possible les détails des distributions en énergie, en position et en direction, déterminant ainsi la précision de la source compacte.

*Maillage adaptatif régularisé.*

Cette méthode de maillage est inspirée de celle proposée par Rozenholc *et al.* Elle sera désignée par l'acronyme « MAP » pour Maximum a posteriori. L'intérêt est de limiter les grandes disparités entre les largeurs des canaux qui peuvent apparaître quand le critère ML seul est utilisé. Dans le cas des fichiers de l'espace des phases PSF faiblement à modérément peuplés, le critère ML est alors sensible à la statistique limitée et peut présenter une tendance à produire un maillage bruité. Cette tendance tend à disparaître avec des PSF fortement peuplés. Afin de proposer un maillage tolérant à une statistique de PSF limitée, le procédé comporte une étape qui régularise les solutions. Le principe de cette méthode consiste à maximiser à nouveau la vraisemblance, mais cette fois-ci, après introduction d'un *a priori* qui favorise des aires de canaux égales, i.e., une répartition en quantiles. L'*a priori* choisi prend ici la forme d'une loi de Dirichlet symétrique par exemple.
D'après le théorème de Bayes, en conservant les notations de l'approche ML, il est possible d'écrire la probabilité de réalisation de la variable X dans l'intervalle I de la façon suivante :

$$P(X,I) = P(I|X)P(X) = P(X|I)P(I) \qquad (4)$$

De cela découle l'expression de la vraisemblance pénalisée $L_2$ :

$$L_2 \triangleq log\big(P(X|I).P(I)\big) \qquad (5)$$

où $P(I)$ désigne *l'a priori* modélisé par une loi de Dirichlet symétrique. Sa densité de probabilité s'exprime de la façon suivante :

$$P(I) \triangleq \frac{\Gamma(D\,\alpha)}{\Gamma(\alpha)^D} \cdot \left(\frac{N_1}{n}\right)^{\alpha-1} \cdot \left(\frac{N_2}{n}\right)^{\alpha-1} \cdots \left(\frac{N_D}{n}\right)^{\alpha-1} \tag{6}$$

où $\alpha$ est un paramètre positif ajustable par l'utilisateur et $\Gamma(x)$ représente la fonction Gamma.
L'expression de la vraisemblance pénalisée est finalement donnée par l'équation (7) :

$$L_2 = \sum_{j=1}^{D} N_j . log\left(\frac{N_j}{n.|I_j|}\right) + (\alpha - 1).\sum_{j=1}^{D} log\left(\frac{N_j}{n}\right) \tag{7}$$

Le choix de valeur de $\alpha$ permet de pondérer l'importance que l'on donne à l'a *priori*. Il est ainsi possible de favoriser un maillage proche des quantiles ou la méthode ML. Si $\alpha$ = 1, le maillage choisi est celui de la méthode ML. Si $\alpha$ est très grand, on se rapproche de celui des quantiles. Typiquement $\alpha$ est choisi égal à 100 par des considérations heuristiques. En effet, l'expression de $L_2$ donnée par l'équation (7) suggère que le paramètre $\alpha$ intervient en substance comme un nombre de particules vis-à-vis de la première somme. Ainsi, il est possible de considérer en première approximation que pour des canaux peuplés avec moins de $\alpha$ (100) particules, l'influence de *l'a priori* est significative.
Le maximum de la vraisemblance pénalisée est obtenu en suivant les mêmes étapes que celles décrites pour l'approche ML. L'unique différence réside dans le calcul de $p_1(i,j)$ à l'étape (a). Pour le maillage régularisé on calcule pour tout *i,j* :

$$p_1(i,j) = \frac{M}{n} \log \frac{M}{n(j-i)\delta} + (\alpha - 1) \log \frac{M}{n}.$$

Une fois le maillage dans chaque dimension réalisé, une partition régularisée de l'espace des phases est obtenue. L'étape suivante consiste à estimer la distribution de probabilité de l'espace des phases pour cette partition fixée.
L'usage est, par exemple, de comptabiliser dans un canal particulier de l'histogramme multidimensionnel les particules dont les valeurs de variables associées sont comprises dans les intervalles correspondants au canal. Cette façon de comptabiliser des particules est connue en statistiques mathématiques comme l'obtention d'un histogramme *empirique.* L'histogramme empirique après normalisation, correspond à l'estimateur par maximum de vraisemblance de la distribution de probabilité de l'espace des phases pour la partition de l'espace donnée (cf. Rozenholc et al. précité). La taille de l'échantillon de particules intervient également ici comme élément limitant de cet estimateur empirique. En effet, chaque canal est alors peuplé suivant la réalisation d'une loi de Poisson dont l'intensité est croissante avec le nombre de particules dans le PSF. Or, plus cette intensité est faible plus le rapport signal/bruit (Moyenne Poisson $m$ = $\lambda$ ; Variance Poisson $\sigma^2 = \lambda$; d'où $\frac{S}{B} = \frac{m}{\sigma} = \sqrt{\lambda}.$ ) du canal en question l'est également. Si les histogrammes 1D correspondant aux lois marginales de chaque grandeur sont largement peuplés, il n'en est pas du tout de même pour une loi jointe en quatre ou cinq dimensions. En effet, dans ce cas, même un espace de phases présentant plus de $10^9$ particules demeure significativement creux, ce qui se traduit par des canaux d'histogrammes multidimensionnels très bruités. Cet effet est une conséquence directe de la grande dimensionnalité associée à la modélisation physique d'un système complexe, tel un accélérateur ou un tube à rayons X.
Il devient donc légitime de considérer, à l'image de la démarche visant à régulariser la taille des canaux rappelée plus haut, la possibilité d'injecter une connaissance a priori sur la forme des distributions d'espaces de phases afin de modérer le bruit induit par la taille limitée du PSF.
Le procédé comporte une étape complémentaire consistant à lisser les histogrammes obtenus afin de limiter les artefacts liées à une statistique limitée se traduisant par des fluctuations aléatoires induites dans les canaux (bruit Poisson). L'étape de lissage du procédé consiste en la réduction de ces fluctuations statistiques dans les canaux du MSC.
La figure 4 illustre le fonctionnement d'un estimateur par lissage par arbre de *Pólya.* L'arbre de *Pólya* est une distribution de probabilité. Pour illustrer de manière simple l'étape de lissage mise en œuvre par le procédé selon l'invention, l'exemple qui suit est donné dans un premier temps, pour le cas d'un *arbre de Pólya monodimensionnel.*
Il est possible d'imaginer un arbre binaire partitionnant l'espace et une collection de nombres positifs $\alpha$ associés à chaque branche de l'arbre. La distribution de l'arbre de Pólya est obtenue en générant récursivement des variables aléatoires de loi Beta paramétrée par les nombres $\alpha$ affectés à chaque sous-branche correspondant à la ramification de la branche de niveau supérieur. La masse de probabilité associée à une branche est répartie aléatoirement sur deux sous-branches. Les notations doivent être choisies afin de pouvoir identifier chaque niveau de ramification de l'arbre.

Pour ce faire, l'indice du canal de la partition correspondant à une branche est représenté par un codage binaire de celui-ci, ce qui permet d'identifier clairement chaque niveau. Ainsi, pour un niveau $m$ particulier, on note $\alpha_{\varepsilon_1 \varepsilon_2 \ldots \varepsilon_m}$ avec $\varepsilon_j = 0$ ou $\varepsilon_j = 1$ le paramètre associé au canal (branche) $k$ noté $\varepsilon_1 \varepsilon_2 \ldots \varepsilon_m$ en codage binaire (à base 2). La ramification de cette branche (découpe aléatoire du canal $B_{\varepsilon_1 \varepsilon_2 \ldots \varepsilon_m}$ en deux sous-parties $B_{\varepsilon_1 \varepsilon_2 \ldots \varepsilon_m 0}$ et $B_{\varepsilon_1 \varepsilon_2 \ldots \varepsilon_m 1}$), est alors obtenue par la génération d'une variable de loi Beta$(\alpha_{\varepsilon_1 \varepsilon_2 \ldots \varepsilon_m 0}, \alpha_{\varepsilon_1 \varepsilon_2 \ldots \varepsilon_m 1})$. La procédure de ramification de l'arbre peut être poursuivie indéfiniment. En pratique, on l'arrête bien entendu à un niveau prédéfini $M$. Il est fréquent de choisir des coefficients $\alpha_{\varepsilon_1 \varepsilon_2 \ldots \varepsilon_m}$ égaux pour un niveau $m$ donné. En particulier, un choix usuel revient à prendre $\alpha_{\varepsilon_1 \varepsilon_2 \ldots \varepsilon_m} = a_m = c\, m^2$ ou $a_m = c\, 2^m$, où $c$ est un nombre positif (typiquement $c = 1$). Cette construction récursive est répétée jusqu'au niveau de ramification maximal souhaité $M$.

Cet *a priori* présente un certain nombre d'avantages. Notamment, l'expression de la loi *a posteriori* de l'arbre de Pólya, prenant en compte les données de l'espace des phases, peut être obtenue de façon analytique. Cet élément est déterminant dans la mesure où une source compacte (en 4D ou plus) fait émerger un nombre de canaux très important, ce qui empêche en pratique de concevoir un algorithme de maximisation en vue d'optimiser un critère statistique de type maximum *a posteriori* (*a minima* de l'ordre de $10^7$ paramètres). De ce fait, l'expression analytique de l'espérance *a posteriori* de la distribution à estimer qui, dans le cas monodimensionnel, peut s'écrire pour le canal $B_{\varepsilon_1 \varepsilon_2 \ldots \varepsilon_m}$ :

$$\mathcal{P}\left(x \in B_{\varepsilon_1 \varepsilon_2 \ldots \varepsilon_m}\right) = \frac{\alpha_{\varepsilon_1} + n_{\varepsilon_1}}{\alpha_0 + \alpha_1 + n} \prod_{j=2}^{m} \frac{\alpha_{\varepsilon_1 \varepsilon_2 \ldots \varepsilon_j} + n_{\varepsilon_1 \varepsilon_2 \ldots \varepsilon_j}}{\alpha_{\varepsilon_1 \varepsilon_2 \ldots \varepsilon_{j-1} 0} + \alpha_{\varepsilon_1 \varepsilon_2 \ldots \varepsilon_{j-1} 1} + n_{\varepsilon_1 \varepsilon_2 \ldots \varepsilon_{j-1}}}$$

où $n_{\varepsilon_1 \varepsilon_2 \ldots \varepsilon_j}$ est le nombre de particules de l'espace des phases présentes dans le canal $B_{\varepsilon_1 \varepsilon_2 \ldots \varepsilon_j}$ et $n$ le nombre total de particules du PSF. On peut remarquer que si tous les coefficients $\alpha$ sont nuls, on trouve

$$\mathcal{P}\left(x \in B_{\varepsilon_1 \varepsilon_2 \ldots \varepsilon_m}\right) = \frac{n_{\varepsilon_1 \varepsilon_2 \ldots \varepsilon_m}}{n}, \ (\ $$

c'est-à-dire l'estimateur ML (empirique) de la source, l'a *priori* n'intervient alors pas.

Dans le cas de sources multidimensionnelles, le passage à un arbre multidimensionnel requiert un certain nombre d'extensions. La modification majeure réside dans le remplacement de la loi Beta par une loi de Dirichlet (voir Annexe) permettant une découpe en plusieurs dimensions [Jara, A.; Hanson, T. E. & Lesaffre, E. Robustifying Generalized Linear Mixed Models Using a New Class of Mixtures of Multivariate Polya Trees, Journal of Computational and Graphical Statistics, 2009, 18, 838-860.]. Comme il a été indiqué, de par sa forte dimensionnalité un modèle histogrammé de source compacte retenant toutes les dimensions de l'espace des phases (4D dans le cas d'une symétrie axiale, 5D sinon) présente un nombre de canaux très important. Par exemple, si on limite en pratique le nombre de canaux à 64 pour chaque variable ($M = 6$ niveaux de ramification par dimension), le nombre de canaux total du modèle de source est alors égal à $64^4 = 16777216$. Dans la suite, l'estimateur de la distribution de l'espace de phases est exprimé pour une dimension arbitraire $K$ et une profondeur d'arbre $M$. Afin de maintenir des expressions relativement compactes, les notations suivantes sont posées: $\varepsilon_{1:m}^{(k)} = \varepsilon_1^{(k)} \varepsilon_2^{(k)} \ldots \varepsilon_m^{(k)}$ pour tout $1 \le m \le M$ et $1 \le k \le K$. Ici, $\varepsilon_{1:m}^{(k)}$ représente l'indice d'un canal de la source dans la dimension $k$. Par conséquent, un canal d'une source à $K$ dimensions sera identifié par les $K$ indices $\varepsilon_{1:m}^{(1)}, \varepsilon_{1:m}^{(2)}, \ldots, \varepsilon_{1:m}^{(K)}$ et sera représenté par $B_{\varepsilon_{1:m}^{(1)}, \varepsilon_{1:m}^{(2)}, \ldots, \varepsilon_{1:m}^{(K)}}$. Les variables binaires $d^{(k)}$ pour $1 \le k \le K$ ($d^{(k)} = 0$ ou $d^{(k)} = 1$) sont également introduites. Il est ainsi possible de construire un indice $\varepsilon_{1:j}^{(k)} = \varepsilon_{1:j-1}^{(k)} d^{(k)}$. À titre d'exemple, l'expression de l'estimateur d'une source monodimensionnelle (1) devient avec ces notations :

$$\mathcal{P}\left(x \in B_{\varepsilon_{1:m}^{(1)}}\right) = \frac{\alpha_{\varepsilon_1^{(1)}} + n_{\varepsilon_1^{(1)}}}{\sum_{d^{(1)}=0}^1 \alpha_{d^{(1)}} + n} \prod_{j=2}^m \frac{\alpha_{\varepsilon_{1:j}^{(1)}} + n_{\varepsilon_{1:j}^{(1)}}}{\sum_{d^{(1)}=0}^1 \alpha_{\varepsilon_{1:j-1}^{(1)} d^{(1)}} + n_{\varepsilon_{1:j-1}^{(1)}}}$$

L'espérance *a posteriori* de l'arbre de Pólya pour une source multidimensionnelle de dimension *K* peut alors s'exprimer :

$$\mathcal{P}\left(x \in B_{\varepsilon_{1:m}^{(1)}, \varepsilon_{1:m}^{(2)}, \dots, \varepsilon_{1:m}^{(K)}}\right)$$

$$= \frac{\alpha_{\varepsilon_1^{(1)}, \varepsilon_1^{(2)}, \dots, \varepsilon_1^{(K)}} + n_{\varepsilon_1^{(1)}, \varepsilon_1^{(2)}, \dots, \varepsilon_1^{(K)}}}{\sum_{d^{(1)}=0}^1 \sum_{d^{(2)}=0}^1 \cdots \sum_{d^{(K)}=0}^1 \alpha_{d^{(1)}, d^{(2)}, \dots, d^{(K)}} + n}$$

$$\times \prod_{j=2}^m \frac{\alpha_{\varepsilon_{1:j}^{(1)}, \varepsilon_{1:j}^{(2)}, \dots, \varepsilon_{1:j}^{(K)}} + n_{\varepsilon_{1:j}^{(1)}, \varepsilon_{1:j}^{(2)}, \dots, \varepsilon_{1:j}^{(K)}}}{\sum_{d^{(1)}=0}^1 \sum_{d^{(2)}=0}^1 \cdots \sum_{d^{(K)}=0}^1 \alpha_{\varepsilon_{1:j-1}^{(1)} d^{(1)}, \varepsilon_{1:j-1}^{(2)} d^{(2)}, \dots, \varepsilon_{1:j-1}^{(K)} d^{(K)}} + n_{\varepsilon_{1:j}^{(1)}, \varepsilon_{1:j}^{(2)}, \dots, \varepsilon_{1:j}^{(K)}}}$$

où $n_{\varepsilon_{1:j}^{(1)}, \varepsilon_{1:j}^{(2)}, \dots, \varepsilon_{1:j}^{(K)}}$ est le nombre de particules de l'espace des phases présentes dans le canal $B_{\varepsilon_{1:j}^{(1)}, \varepsilon_{1:j}^{(2)}, \dots, \varepsilon_{1:j}^{(K)}}$.

Cette expression autorise un calcul rapide de l'estimateur bayésien de source compacte à partir de l'histogramme 4D des particules de l'espace des phases. Ceci peut être interprété comme un *post filtrage non linéaire* des données histogrammées.
Un choix de paramètres tel que :

$$\alpha_{\varepsilon_{1:m}^{(1)}, \varepsilon_{1:m}^{(2)}, \dots, \varepsilon_{1:m}^{(K)}}$$

$$= a_m \, 2^K \frac{n_{\varepsilon_{1:m}^{(1)}} n_{\varepsilon_{1:m}^{(2)}} \dots n_{\varepsilon_{1:m}^{(K)}}}{\sum_{d^{(1)}=0}^1 \sum_{d^{(2)}=0}^1 \cdots \sum_{d^{(K)}=0}^1 n_{\varepsilon_{1:m-1}^{(1)} d^{(1)}} n_{\varepsilon_{1:m-1}^{(2)} d^{(2)}} \dots n_{\varepsilon_{1:m-1}^{(K)} d^{(K)}}}$$

permet de *centrer* l'arbre sur la loi correspondant au produit des lois marginales une dimension, 1D, des *K* variables. Ce paramétrage se révèle intéressant notamment quand les différentes variables de la source sont faiblement corrélées. L'arbre de Pólya n'intervient alors que comme une correction multidimensionnelle conjointe par rapport à une situation où les variables de l'espace de phases seraient considérées indépendantes (à la façon d'une fonction *copule*).

Une autre solution est dite *Arbre de Pólya « adouci »*. L'estimateur de la distribution d'espace de phases présenté ci-dessus est continu en tout point sauf aux frontières de la partition où il peut présenter des discontinuités importantes. Pour pallier ce phénomène, il est possible d'imaginer un modèle de source compacte où la partition n'est plus considérée comme *unique* mais *distribuée* de façon à tempérer ces effets de discontinuités. Une manière de procéder consiste à décaler l'origine de la partition multidimensionnelle suivant tous les décalages ou offsets possibles (soit $M^K$ offsets possibles en dimension *K*) et de procéder au calcul de l'espérance *a posteriori* de l'arbre pour chaque décalage. Afin de conserver un *a priori* identique garantissant la même distribution centrale quel que soit le décalage, les coefficients $\alpha_{\varepsilon_{1:m}^{(1)}, \varepsilon_{1:m}^{(2)}, \dots, \varepsilon_{1:m}^{(K)}}$ doivent être modifiés de façon *ad hoc*. Afin d'expliciter cette recombinaison nous notons $\delta_{1:M}^{(k)}$ le décalage de la partition pour la dimension *k* au niveau *M* le plus ramifié de l'arbre. Dans la partition décalée, l'indice en codage binaire du canal initial $\varepsilon_{1:M}^{(k)}$ devient

$$(\varepsilon_{1:M}^{(k)} + \delta_{1:M}^{(k)}) \bmod 2^M = \tilde{\varepsilon}_{1:M}^{(k)}$$

Cette rotation de l'indice suppose implicitement que la distribution marginale de chaque variable présente des valeurs comparables pour les bornes minimum et maximum de son support. Ceci est vérifié intrinsèquement pour les angles et observé pour les variables rayon et énergie.

Pour *chaque décalage en K dimensions* $\Delta = \delta_{1:M}^{(1)}, \delta_{1:M}^{(2)} ..., \delta_{1:M}^{(K)}$, on reconstruit un histogramme empirique pour les canaux $B_{\tilde{\varepsilon}_{1:M}^{(1)}, \tilde{\varepsilon}_{1:M}^{(2)},...,\tilde{\varepsilon}_{1:M}^{(K)}}$ de la partition décalée où le nombre de particules de l'espace des phases est alors noté $n_{\tilde{\varepsilon}_{1:M}^{(1)}, \tilde{\varepsilon}_{1:M}^{(2)},...,\tilde{\varepsilon}_{1:M}^{(K)}}^{\Delta}$. De la même façon, $n_{\tilde{\varepsilon}_{1:M}^{(k)}}^{\Delta}$ représente le nombre de particules de la marginale *k* dans la partition étendue. En remontant l'arbre jusqu'à sa racine, on peut évaluer $n_{\tilde{\varepsilon}_{1:m}^{(1)}, \tilde{\varepsilon}_{1:m}^{(2)},...,\tilde{\varepsilon}_{1:m}^{(K)}}^{\Delta}$ et $n_{\tilde{\varepsilon}_{1:m}^{(k)}}^{\Delta}$ pour tout *m*. Dès lors, les coefficients de l'arbre permettant de garantir un *a priori* identique quel que soit $\Delta$ s'écrivent :

$$\alpha_{\tilde{\varepsilon}_{1:m}^{(1)}, \tilde{\varepsilon}_{1:m}^{(2)},...,\tilde{\varepsilon}_{1:m}^{(K)}}^{\Delta}$$

$$= a_m \, 2^K \frac{n_{\tilde{\varepsilon}_{1:m}^{(1)}}^{\Delta} \, n_{\tilde{\varepsilon}_{1:m}^{(2)}}^{\Delta} \, ... \, n_{\tilde{\varepsilon}_{1:m}^{(K)}}^{\Delta}}{\sum_{d^{(1)}=0}^{1} \sum_{d^{(2)}=0}^{1} \cdots \sum_{d^{(K)}=0}^{1} n_{\tilde{\varepsilon}_{1:m-1}^{(1)} d^{(1)}}^{\Delta} \, n_{\tilde{\varepsilon}_{1:m-1}^{(2)} d^{(2)}}^{\Delta} \, ... \, n_{\tilde{\varepsilon}_{1:m-1}^{(K)} d^{(K)}}^{\Delta}}$$

Il suffit alors de substituer $\alpha_{\tilde{\varepsilon}_{1:j}^{(1)}, \tilde{\varepsilon}_{1:j}^{(2)},...,\tilde{\varepsilon}_{1:j}^{(K)}}^{\Delta}$ à $\alpha_{\varepsilon_{1:j}^{(1)}, \varepsilon_{1:j}^{(2)},...,\varepsilon_{1:j}^{(K)}}$ et $n_{\tilde{\varepsilon}_{1:j}^{(1)}, \tilde{\varepsilon}_{1:j}^{(2)},...,\tilde{\varepsilon}_{1:j}^{(K)}}^{\Delta}$ à $n_{\varepsilon_{1:j}^{(1)}, \varepsilon_{1:j}^{(2)},...,\varepsilon_{1:j}^{(K)}}$ dans la formule de l'espérance *a posteriori* de la distribution multidimensionnelle d'espace des phases pour obtenir l'expression de

$$\mathcal{P}\left(x^{\Delta} \in B_{\tilde{\varepsilon}_{1:M}^{(1)}, \tilde{\varepsilon}_{1:M}^{(2)},...,\tilde{\varepsilon}_{1:M}^{(K)}}\right)$$

où $x^{\Delta}$ représente le point *x* de l'espace des phases décalé de $\Delta$. On note

$$\mathcal{P}^{\Delta}\left(x \in B_{\varepsilon_{1:M}^{(1)}, \varepsilon_{1:M}^{(2)},...,\varepsilon_{1:M}^{(K)}}\right) = \mathcal{P}\left(x^{\Delta} \in B_{\tilde{\varepsilon}_{1:M}^{(1)}, \tilde{\varepsilon}_{1:M}^{(2)},...,\tilde{\varepsilon}_{1:M}^{(K)}}\right)$$

la distribution ainsi obtenue après suppression de l'offset $\Delta$ (recalage).

L'estimateur « adouci » est obtenu en prenant la moyenne des espérances a *posteriori* pour tous les décalages $\Delta$ considérés. Avec cette construction, il est possible de prendre en compte $M^K$ valeurs distinctes de décalages, ce qui n'est pas réaliste d'un point de vue pratique pour une source 4D. On se restreint alors à quelques dizaines de décalages.

$$\bar{\mathcal{P}}\left(x \in B_{\varepsilon_{1:M}^{(1)}, \varepsilon_{1:M}^{(2)},...,\varepsilon_{1:M}^{(K)}}\right) = \sum_{\Delta} \mathcal{P}^{\Delta}\left(x \in B_{\varepsilon_{1:M}^{(1)}, \varepsilon_{1:M}^{(2)},...,\varepsilon_{1:M}^{(K)}}\right)$$

Applications étudiées en radiothérapie et imagerie :

- Modèles de sources pour des accélérateurs linéaires médicaux, intégrables aux logiciels utilisant ces modèles (treatment planning system, TPS),
- Modèles de sources pour des tubes à rayons X équipant des systèmes d'imagerie RX 2D ou 3D,
- Modèles prédictifs de type MC pour les imageurs planaires de type flat-panel (EPID, systèmes d'imagerie cone-beam CT),
- En radiothérapie : dosimétrie hors-champ, aux organes à risque et aux tissus sains recevant une faible dose.

Le procédé et le système selon l'invention permettent notamment de réduire de l'ordre de trois décades la quantité d'information nécessaire à stocker tout en restituant des caractéristiques de faisceau moins sensibles au bruit d'origine statistique.

**Revendications**

1. Procédé exécuté par un processeur pour estimer les paramètres caractéristiques physiques ou variables d'un faisceau de corpuscules destiné à être émis vers un corps ou un objet et pour déterminer la dose déposée par le faisceau émis, les corpuscules étant rangés dans un fichier d'espace des phases, comportant en combinaison au moins les étapes suivantes :

   • Collecter les corpuscules présents dans un faisceau ayant traversé un collecteur (23) et un filtre (24), ayant interagi ou non avec eux,
   • Déterminer une partition d'un support $\Omega$ de chaque variable sous forme d'un ensemble $I$ d'intervalles consécutifs et contigus pour le classement des corpuscules du fichier PSF en énergie, position et direction, la réunion de ces intervalles pour chaque variable du faisceau constituant un canal multi-varié, et

   **Caractérisé en ce qu'**il comporte les étapes suivantes :

   • Découper ledit support $\Omega$ en un nombre Dmax de micro-canaux réguliers de taille donnée $\delta$ et calculer les vraisemblances correspondant aux intervalles possibles par concaténation contigüe des micro-canaux, retenir la meilleure partition $I$ de $\Omega$,
   • Définir un maillage dans chaque direction de l'espace des phases en maximisant la vraisemblance de la réalisation d'une variable dans un intervalle en introduisant un « a priori », afin d'obtenir une partition régularisée de l'espace des phases,
   • Estimer la distribution de probabilité de l'espace des phases pour la partition régularisée,
   • Calculer les caractéristiques physiques du faisceau de corpuscules, les comparer à des données prévues et déterminer les doses avant d'émettre le faisceau.

2. Procédé selon la revendication 1 **caractérisé en ce que** pour définir le maillage on exécute les étapes suivantes :

   (a) découper le support $\Omega$ en un grand nombre $D_{max}$ de micro-canaux réguliers de tailles égales à $\delta$,
   (b) associer ces micro-canaux selon tous les regroupements contigus possibles à chaque fois de manière à produire $D$ canaux,
   (c) déterminer la partition $I$ de $\Omega$, qui maximise la valeur $L_1$. log vraisemblance des intervalles en fonction des corpuscules du fichier PSF $L_1 = \sum_{j=1}^{D} N_j . \log\left(\frac{N_j}{n|I_j|}\right),$ $L_1$ où $D$ est le nombre de canaux du fichier PSF, $N_j$ le nombre de corpuscules observables du canal $j$, $I_j$ l'intervalle pour le canal $j$, $n$ le nombre total de corpuscules dans le faisceau.

3. Procédé selon la revendication 2 **caractérisé en ce qu'**il comporte pour optimiser la valeur $L_1$ au moins les étapes suivantes :

   *Étape (a) :*
   Pour $i = 0 ... D_{max}$ $D_{max}$ : nombre de micro-canaux donnés initialement
   Initialiser $M = 0$
   Pour $j = i + 1 ... D_{max}$ boucle sur les micro-canaux j, j>i,

   Calculer $M = M + m_i$ où $m_i$ représente le nombre de particules dans le micro-canal $I$.
   Calculer $p_1(i,j) = \frac{M}{n} \log \frac{M}{n(j-i)\delta}$ probabilité de regrouper les micro-canaux de i à j pour former un canal,

   *Étape (b) :*

   Initialiser pour $j = 1 ... D_{max}$ boucle sur les j micro-canaux $q(j) = p_1(0,j)$

Pour $i = 2 \dots D$ (nombre de canaux du PSF)

Pour $j = i \dots D_{max}$ boucle sur les j micro-canaux
Initialiser $S = -\infty$
Pour $d = i - 1 \dots j - 1$

$$s = q(d) + p_1(d,j)$$

Si $s > S$
$S = s$
$H(i,j) = d$

$$p(i,j) = q\big(H(i,j)\big) + p_1(H(i,j),j)$$

Pour $j = i \dots D_{max}$ boucle sur les j micro-canaux
$q(j) = p(i,j)$

*Étape (c)* :

Initialiser $L = D_{max}$ $D_{max}$ : nombre de micro-canaux donnés initialement
Pour $j = D \dots 1$
$L = c(H(j,L[1]),L)$ où $L[1]$ représente la première coordonnée du vecteur $L$
et $c(u, L)$ la concaténation des vecteurs $u$ et $L$,
on obtient le partitionnement des intervalles.

4. Procédé selon l'une des revendications 1 à 3 **caractérisé en ce que** l'on utilise une loi de Dirichlet symétrique comme « a priori ».

5. Procédé selon l'une des revendications 1 à 4 **caractérisé en ce que** pour estimer la distribution de probabilité des corpuscules de l'espace des phases PSF pour une partition régularisée, on comptabilise dans un canal particulier de l'histogramme multidimensionnel formé par intervalles pour les différentes variables, énergie, position, direction des corpuscules, les corpuscules dont les valeurs de variables associées sont comprises dans les intervalles correspondants.

6. Procédé selon la revendication 5 **caractérisé en ce qu'**il comporte une étape de lissage des histogrammes obtenus en utilisant un arbre de Pólya multidimensionnel et en exprimant la loi a posteriori de l'arbre de Pólya selon une expression analytique.

7. Procédé selon l'une des revendications précédentes **caractérisé en ce que** l'on détermine une dose déposée par des rayons X d'un dispositif médical.

8. Dispositif pour estimer les paramètres caractéristiques physiques d'un faisceau de corpuscules destiné à être émis vers un corps ou un objet et pour déterminer la dose à émettre, les corpuscules étant rangés dans un fichier d'espace des phases, **caractérisé en ce qu'**il comporte au moins les éléments suivants :

• Un collecteur (23) des corpuscules émis dans un faisceau,
• Un processeur (27) adapté à exécuter les étapes du procédé selon l'une des revendications 1 à 7.

9. Dispositif selon la revendication 8 **caractérisé en ce que** le dispositif est un dispositif médical et les corpuscules des rayons X.

**Patentansprüche**

1. Verfahren, ausgeführt von einem Prozessor zum Schätzen der physikalischen oder variablen charakteristischen Parameter eines Strahls von Korpuskeln, bestimmt zum Emittieren zu einem Körper oder einem Objekt und zum Bestimmen der von dem emittierten Strahl abgesetzten Dosis, wobei die Korpuskeln in einer Phasenraumdatei angeordnet sind, das in Kombination wenigstens die folgenden Schritte beinhaltet:

• Sammeln der in einem Strahl vorhandenen Korpuskeln, die einen Kollektor (23) und ein Filter (24) durchlaufen haben, ob sie damit interagiert haben oder nicht,
• Bestimmen einer Partition eines Trägers $\Omega$ jeder Variablen in Form eines Satzes $I$ von aufeinander folgenden und zusammenhängenden Intervallen zum Klassifizieren der Korpuskeln der PSF-Datei in Energie, Position und Richtung, wobei die Vereinigung dieser Intervalle für jede Variable des Strahls einen multivariaten Kanal bildet, und

**dadurch gekennzeichnet, dass** es die folgenden Schritte beinhaltet:

• Unterteilen des Trägers $\Omega$ in eine Anzahl Dmax von regelmäßigen Mikrokanälen einer gegebenen Größe $\delta$ und Berechnen der Plausibilitäten entsprechend den möglichen Intervallen durch zusammenhängende Verkettung der Mikrokanäle, Behalten der besten Partition $I$ von $\Omega$,
• Definieren eines Netzes in jeder Richtung des Phasenraums durch Maximieren der Plausibilität der Realisierung einer Variablen in einem Intervall durch Einführen eines "a priori", um eine regulierte Partition des Phasenraums zu erhalten,
• Schätzen der Wahrscheinlichkeitsverteilung des Phasenraums für die regulierte Partition,
• Berechnen der physikalischen Charakteristiken des Korpuskelstrahls, Vergleichen derselben mit vorgesehenen Daten und Bestimmen der Dosen vor dem Emittieren des Strahls.

2. Verfahren nach Anspruch 1, **dadurch gekennzeichnet, dass** zum Definieren des Netzes die folgenden Schritte durchgeführt werden:

(a) Unterteilen des Trägers $\Omega$ in eine große Zahl $D_{max}$ von regelmäßigen Mikrokanälen mit Größen gleich $\delta$,
(b) Assoziieren der Mikrokanäle gemäß allen möglichen zusammenhängenden Gruppierungen jedes Mal so, dass $D$ Kanäle entstehen,
(c) Bestimmen der Partition $I$ von $\Omega$, die den Wert $L_1$ maximiert, die log-Wahrscheinlichkeit der Intervalle in

$$L_1 = \sum_{j=1}^{D} N_j . \log\left(\frac{N_j}{n|I_j|}\right),$$

Abhängigkeit von den Korpuskeln der PSF-Datei, wobei $D$ die Anzahl der Kanäle der PSF-Datei ist, $N_j$ die Anzahl von beobachtbaren Korpuskeln des Kanals $j$ ist, $I_j$ das Intervall für den Kanal $j$ ist, $n$ die Gesamtzahl der Korpuskeln in dem Strahl ist.

3. Verfahren nach Anspruch 2, **dadurch gekennzeichnet, dass** es zum Optimieren des Wertes $L_1$ wenigstens die folgenden Schritte beinhaltet:

*Schritt (a):*
für $i = 0 ... D_{max}$ $D_{max}$ : Anzahl von anfangs gegebenen Mikrokanälen
Initialisieren von $M = 0$
für $j = i + 1 ... D_{max}$ Schleife auf den Mikrokanälen j, j>i,

Berechnen von $M = M + m_i$, wobei $m_I$ die Anzahl von Partikeln im Mikrokanal $I$ repräsentiert;

$$p_1(i,j) = \frac{M}{n} \log \frac{M}{n(j-i)\delta},$$

Berechnen von Wahrscheinlichkeit des Gruppierens der Mikrokanäle i bis j zum Bilden eines Kanals,

*Schritt (b):*

Initialisieren für $j = 1 ... D_{max}$ Schleife auf den j Mikrokanälen
$q(j) = p_1(0,j)$
für $i = 2 ... D$ (Anzahl Kanäle des PSF)

für $j = i ... D_{max}$ Schleife auf den j Mikrokanälen
Initialisieren von $S = -\infty$
für $d = i - 1 ... j - 1$

$$s = q(d) + p_1(d,j)$$

wenn $s > S$
$S = s$
$H(i,j) = d$

$$p(i,j) = q(H(i,j)) + p_1(H(i,j),j)$$

für $j = i \ldots D_{max}$ Schleife auf den j Mikrokanälen
$q(j) = p(i,j)$

*Schritt (c):*
Initialisieren von $L = D_{max}$ $D_{max}$ : Anzahl von anfangs gegebenen Mikrokanälen für $j = D \ldots 1$

$L = c(H(j,L[1]),L)$, wobei $L[1]$ die erste Koordinate des Vektors $L$ repräsentiert und $c(u,L)$ die Verkettung der Vektoren $u$ und $L$ repräsentiert,
die Partitionierung der Intervalle erhalten wird.

4. Verfahren nach einem der Ansprüche 1 bis 3, **dadurch gekennzeichnet, dass** ein symmetrisches Dirichlet-Gesetz wie "a priori" benutzt wird.

5. Verfahren nach einem der Ansprüche 1 bis 4, **dadurch gekennzeichnet, dass** zum Schätzen der Wahrscheinlichkeitsverteilung von Korpuskeln des PSF-Phasenraums für eine regulierte Partition in einem bestimmten Kanal des mehrdimensionalen Histogramms, gebildet durch Intervalle für die unterschiedlichen Variablen Energie, Position, Richtung von Korpuskeln, die Korpuskeln gezählt werden, deren assoziierte Variablenwerte in den entsprechenden Intervallen enthalten sind.

6. Verfahren nach Anspruch 5, **dadurch gekennzeichnet, dass** es einen Schritt des Glättens der erhaltenen Histogramme durch Benutzen eines mehrdimensionalen Polya-Baums und durch Ausdrücken des a posteriori Gesetzes des Polya-Baums gemäß einem analytischen Ausdruck beinhaltet.

7. Verfahren nach einem der vorherigen Ansprüche, **dadurch gekennzeichnet, dass** eine durch die Röntgenstrahlen einer medizinischen Vorrichtung abgesetzte Dosis bestimmt wird.

8. Vorrichtung zum Schätzen der physikalischen charakteristischen Parameter eines Strahls von Korpuskeln, bestimmt zum Emittieren zu einem Körper oder einem Objekt und zum Bestimmen der zu emittierenden Dosis, wobei die Korpuskeln in einer Phasenraumdatei angeordnet sind, **dadurch gekennzeichnet, dass** sie wenigstens die folgenden Elemente umfasst:

   • einen Kollektor (23) für in einem Strahl emittierte Korpuskeln,
   • einen Prozessor (27), ausgelegt zum Durchführen der Schritte des Verfahrens nach einem der Ansprüche 1 bis 7.

9. Vorrichtung nach Anspruch 8, **dadurch gekennzeichnet, dass** die Vorrichtung eine medizinische Vorrichtung ist und die Korpuskeln Röntgenstrahlen sind.

## Claims

1. Process carried out by a processor in order to estimate the physical or variable characteristic parameters of a bundle of corpuscles which are intended to be emitted towards a body or an object and in order to determine the dose deposited by the bundle emitted, the corpuscles being arranged in a phase space register, comprising in combination at least the following steps:

   • collecting the corpuscles present in a bundle which has passed through a collector (23) and a filter (24) and which may or may not have interacted therewith,

• determining a partition of a support $\Omega$ of each variable in the form of a set $I$ of consecutive and contiguous ranges for classifying the corpuscles of the register PSF in terms of energy, position and direction, the reassembly of these ranges for each variable of the bundle constituting a multivariate channel, and

**characterised in that** it comprises the following steps:

• cutting the support $\Omega$ into a number Dmax of regular micro-channels having a given size $\delta$ and calculating the likelihood corresponding to the possible ranges by contiguous concatenation of the micro-channels, retaining the best partition I of $\Omega$,
• defining a link in each direction of phase space while maximising the probability of the construction of a variable in a range while introducing one "a priori" in order to obtain a regularised partition of the phase space,
• estimating the probability distribution of the phase space for the regularised partition,
• calculating the physical characteristics of the bundle of corpuscles, comparing them with proven data and determining the doses before emitting the bundle.

2. Process according to claim 1, **characterised in that**, in order to define the link, the following steps are carried out:

(a) cutting the support $\Omega$ into a large number $D_{max}$ of regular micro-channels having sizes of $\delta$,
(b) associating these micro-channels in accordance with all the possible contiguous groups each time in order to produce D channels,
(c) determining the partition 1 of $\Omega$ which maximises the value $L_1$. log likelihood of the ranges as a function of

$$L_1 = \sum_{j=1}^{D} N_j . \log\left(\frac{N_j}{n|I_j|}\right)$$

the corpuscles of the register PSF, where D is the number of channels of the register PSF, $N_j$ is the number of corpuscles of the channel j which can be observed, Ij is the range for the channel j, n is the total number of corpuscles in the bundle.

3. Process according to claim 2, **characterised in that** it comprises, in order to optimise the value $L_1$. at least the following steps:

Step (a):
for $i = 0 ... D_{max}$ , $D_{max}$: number of micro-channels given initially

initialise $M = 0$
for $j = i + 1 ... D_{max}$, loop on the micro-channels j, j>i,

calculate $M = M + m_i$ where $m_l$ represents the number of particles in the micro-channel 1,

$$p_1(i,j) = \frac{M}{n} \log \frac{M}{n(j-i)\delta}$$

calculate probability of reassembling the micro-channels from i to j in order to form a channel,

Step (b):
initialise for $j = 1 ... D_{max}$ loop on the j micro-channels
$q(j) = p_1(0,j)$ for i = 2 ... D (number of channels of PSF)
for $j = i ... D_{max}$ loop on the j micro-channels
initialise S = $-\infty$
for d = i-1 ... j-1

$$s = q(d) + p_1(d,j)$$

Si $s > S$
$S = s$
$H(i,j) = d$

$$p(i,j) = q\big(H(i,j)\big) + p_1(H(i,j),j)$$

For $j = i \ldots D_{max}$ loop on the j micro channels

$q(j) = p(i,j)$

<u>Step (c):</u>

initialise L = $D_{max}$, $D_{max}$: number of micro-channels given initially

for $j = D \ldots 1$

$L = c(H(j,L[1]),L)$ where $L$[1] represents the first coordinate of the vector $L$, and $c(u, L)$ the concatenation of the vectors $u$ and $L$,

there is obtained the distribution of the ranges.

4. Process according to any one of claims 1 to 3, **characterised in that** a symmetrical Dirichlet law is used as "a priori".

5. Process according to any one of claims 1 to 4, **characterised in that**, in order to estimate the distribution of probability of the corpuscles of the phase space PSF for a regularised partition, there is recorded in a specific channel of the multi-dimensional histogram formed by ranges for the different variables, energy, position, direction of the corpuscles, the corpuscles, of which the values of associated variables are contained in the corresponding ranges.

6. Process according to claim 5, **characterised in that** it comprises a step of smoothing the histograms obtained using a multi-dimensional Polya tree and by expressing the law a posteriori of the Polya tree according to an analytical expression.

7. Process according to any one of the preceding claims, **characterised in that** a dose deposited by the X-rays of a medical device is determined.

8. Device for estimating the physical characteristic parameters of a bundle of corpuscles which is intended to be emitted towards a body or an object and in order to determine the dose to be emitted, the corpuscles being arranged in a phase space register, **characterised in that** it comprises at least the following steps:

   • a collector (23) of the corpuscles emitted in a bundle,
   • a processor (27) which is suitable for carrying out the steps of the process according to any one of claims 1 to 7.

9. Device according to claim 8, **characterised in that** the device is a medical device and the corpuscles are X-rays.

$\varphi_{rel} = \varphi_s - \varphi_0$

$(x_0, y_0) = (r_0, \varphi_0)$

Plan origine de la source

$(x, y, u, v)$

Plan du PSF

$(x_s, y_s) = (r_s, \varphi_s)$

Plan d'échantillonnage

# FIG.1

Vers module de régulation du faisceau à émettre

27

PSF

# FIG.2

FIG.3

FIG.4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6535837 B

**Littérature non-brevet citée dans la description**

- **C.M. MA ; B.A. FADDEGON ; D.W.O. ROGERS ; T.R. MACKIE.** Accurate characterization of Monte Carlo calculated electron beams for radiotherapy. *Medical Physics,* 1997, vol. 24, 401-416
- **A.E. SCHACH VON WITTENAU et al.** Correlated Histogram Representation of Monte Carlo Derived Medical Accelerator Photon-Output Phase Space. *Medical Physics,* 1999, vol. 26 (7), 1196-1211
- **M. FIX et al.** Monte Carlo Source Model for Megavoltage Photon-Beam Radiotherapy: Photon Source Characteristics. *Medical Physics,* 2004, vol. 31 (11), 3106-3121

- **DENG et al.** Photon Beam Characterization and Modelling for Monte Carlo Treatment Planning. *Physics in Medicine and Biology,* 2000, vol. 45 (2), 411-427
- **ROZENHOLC et al.** Combining regular and irregular histograms by penalized likelihood. *Computational Statistics and Data Analysis,* 2010, vol. 54, 3313-3323
- **JARA, A. ; HANSON, T. E. ; LESAFFRE, E.** Robustifying Generalized Linear Mixed Models Using a New Class of Mixtures of Multivariate Polya Trees. *Journal of Computational and Graphical Statistics,* 2009, vol. 18, 838-860